# EUROPEAN PATENT SPECIFICATION

(11) **EP 1 185 686 B1**
(45) Date of publication and mention of the grant of the patent: **17.08.2005**
(21) Application number: 00935749.2
(22) Date of filing: 31.05.2000
(51) Int. Cl.: C12Q 1/04

(54) **CULTURE MEDIUM FOR DETECTION OF DEKKERA AND BRETTANOMYCES**
KULTURMEDIUM ZUM NACHWEIS VON DEKKERA UND BRETTANOMYCES
MILIEU DE CULTURE DE DETECTION DE DEKKERA ET BRETTANOMYCES

(30) Priority: 31.05.1999 PT 10230699
(43) Date of publication of application: 13.03.2002
(73) Proprietor: Stab Vida-Investigacao e Servicos EM Ciencias Biologicas, Lda., 2781-601 Oeiras (PT); Instituto Superior De Agronomia, 1399 Lisboa (PT)
(72) Inventor: LOUREIRO, Virgilio Borges, P-1070 Lisboa (PT); GONCALVES, Maria da Graça Alves;, P-2000-647 Santarém (PT); RODRIGUES, Nuno Miguel Sousa Falcao Freire, P-1700-076 Lisboa (PT)
(86) International application number: PCT/PT2000/000005
(87) International publication number: WO 2000/073495

(56) References cited:
- P. CHATONNET ET AL.: "The origin of ethylphenols in wines" JOURNAL OF THE SCIENCE OF FOOD AND AGRICULTURE., vol. 60, no. 2, 1992, pages 165-178, XP002148620 ELSEVIER APPLIED SCIENCE PUBLISHERS. BARKING., GB ISSN: 0022-5142 cited in the application
- CHEMICAL ABSTRACTS, vol. 122, no. 9, 27 February 1995 (1995-02-27) Columbus, Ohio, US; abstract no. 101322, D. A. N. EDLIN ET AL.: "The biotransformation of simple phenolic compounds by Brettanomyces anomalus" page 574; column 1; XP002148621 & FEMS MICROBIOL. LETT., vol. 125, no. 2-3, 1995, pages 311-316,
- CHEMICAL ABSTRACTS, vol. 106, no. 3, 19 January 1987 (1987-01-19) Columbus, Ohio, US; abstract no. 15531, T. HERESZTYM: "Metabolism of volatile phenolic compounds from hydroxycinnamic acids by Brettanomyces yeast" page 331; column 1; XP002148622 & ARCH. MICROBIOL., vol. 146, no. 1, 1986, pages 96-98,

## Description

### Object of the Invention

The present invention refers to a culture medium for the differential detection and enumeration of food and beverage contaminant yeasts of the *Dekkera* and *Brettanomyces* genera, containing ethanol and *p*-cumaric acid in its preparation. It is also an object of the present invention a method using said culture medium for the differential detection and enumeration of yeasts of the *Dekkera* and *Brettanomyces* genera. It is still an object of the present invention the use of said culture medium in a yeast identification gallery.

The objective of the present invention consists in providing the food and beverage industry with a culture medium and a method using said culture medium, able to isolate, differentially detect and number yeasts of the *Dekkera* and *Brettanomyces* genera, by means of the color changing of the culture medium and of the colonies therein developed, and production of a characteristic phenol-like aroma.

### State of the Art

The study and characterization of the yeast microflora present in many different habitats (e.g. beverages, food, natural substracts) generally involves a first strain isolation and purifying stage in generic yeast culture media, which is followed by a second identification stage by classical taxonomic methods or by molecular biology based techniques. The slow development of the yeasts of the *Dekkera* and *Brettanomyces* genera makes its isolation in the commonly used media extremely difficult, since these yeasts are overtaken by faster developing ones that coexist with them. This fact makes their detection and enumeration, as well as their later identification in food and beverages, rather difficult, this being generally accomplished through the use of very slow, work intensive, and technical skill demanding classical techniques or through molecular biology techniques, involving the use of expensive reactants, molecular probes or primers not always promptly available in the market, and of skilled operators.

It was possible to establish beyond any doubt that these yeasts are involved in the production of a serious organoleptic defect in wines - "horse sweat" - particularly in those that are aged in oak casks. (Chatonnet, *et al*. 1992, *J. Sc. Food Agric.,* 60,165-178). Since then, their detection and enumeration in wines became essential, arising the need for the development of swift methods for that effect. The field bibliography discloses suitable means for the detection and enumeration of yeasts of the *Dekkera* and *Brettanomyces* genera based on this species resistance to cycloheximide and their acidifying ability (Chatonnet, *et al*. 1992, *J. Sc. Food Agric.,* 60,165-178; Fugelsang, K. *et al.* 1993. Ed. Barry H. Gump. ACS Symposium series 536, *American Chemical Society,* Washington. Cap. 7, 110-119; Alguacil, M. *et al.* 1998. *Aliment. Equipos Tecnol,* 10, 81-85). However, the disclosed.media were not entirely satisfactory, since they were not selective enough to prevent the growing of fast developing species, and also were not totally differential.

Therefore, there is a real and effective need for a culture medium and method for the easy and swift identification of yeasts of the *Dekkera* and *Brettanomyces* genera, namely to provide the food and beverage industry a swift method for the isolation, differential detection arid enumeration of yeasts of the *Dekkera* and *Brettanomyces* genera.

### Description of the Invention

It was surprisingly found that using a culture medium containing ethanol as the only energy source and *p*-cumaric acid for *Dekkera* and *Brettanomyces* genera yeasts growth, these produce 4-ethylphenol and acetic acid in a characteristic and exclusive form, compared to other yeasts, after incubation for 5 to 12 days. Furthermore, other species of yeasts, usually coexistent with the yeasts of the *Dekkera* and *Brettanomyces* genera, which much faster development disallow their detection, are selectively inhibited.

According to the invention, a culture medium was developed, which is partially selective and totally differential for yeasts of the *Dekkera* and *Brettanomyces* genera, which is based on 4-ethylphenol detection, by its characteristic aroma, and acetic acid, by means of an adequate acid-base color change, produced by those yeasts when developed in a ethanol and *p*-cumaric acid containing medium.

Therefore, the present invention refers to a culture medium for the differential detection and enumeration of food and beverage contaminant yeasts of the *Dekkera* and *Brettanomyces* genera. Said medium comprises a nutrient base, ethanol as a non-fermentable energy source and inhibiting other yeasts species, *p*-cumaric acid as an aromatic compound promoting substract produced by said yeasts species, an acid-base indicator whose turning points lie within the acid range (particularly bromocresol green), an antibiotic that inhibits several species of yeasts (particularly cycloheximide), and agar-agar when the medium is intended for use in a solid form.

In an embodiment, in the culture medium according to the invention the nutrient base is "Yeast Nitrogen Base" and the acid-base indicator is bromocresol green.

In another embodiment, the culture medium according to the invention further contains a bacterial growth inhibitor, particularly chloramphenicol and/or oxytetracycline, which is specially useful for the detection of yeasts of the *Dekkera* and *Brettanomyces* genera within mixed bacteria including populations.

The medium object of the invention, when in the liquid form, is prepared by sterilizing filtration, then being dispensed into adequate test tubes. When the medium is desired in the solid form, agar-agar is dissolved in demineralized water and then sterilized in an autoclave; following the sterilization and cooling to about 50°C, the other components are added under aseptic conditions, having previously been filtration sterilized. The pH adjustment for both media is done before the sterilization, to a pH value that depends on the acid-base indicator used. The mixture is homogenized and poured into Petri dishes, the medium being ready for use after solidifying.

The present invention also refers to a method for the detection and enumeration of yeasts of the *Dekkera* and *Brettanomyces* genera, using a partially selective and totally differential culture medium, characterized as above.

The method according to the invention allows the detection and enumeration of yeasts of the *Dekkera* and *Brettanomyces* genera in a simple way using the culture medium according to the present invention.

According to the invention, the culture medium is used by direct contact with a sample under analysis, adequate dilutions being used if the enumeration of the contaminant yeasts is intended. The thus inoculated medium is then incubated at an adequate temperature for the growth of the yeasts, during a time period enough, usually 5 to 10 days, for the development of clearly visible colonies (in case of the solid medium) or for the clouding of the solution (in case of the liquid medium). The detection of the yeasts of the *Dekkera* and *Brettanomyces* genera in the Petri dishes is accomplished by direct observation of cream colored colonies, by the change of medium color according to the type of acid-base indicator that was used, and by the presence of a characteristic phenol-like aroma. If the incubation time is extended, the colonies acquire a darker coloring. The detection in the liquid medium is accomplished by the change of the medium color and by the presence of the phenol-like aroma.

In addition, it is a convenient, swift, and easily reproducible procedure by any microbiological culture media manufacturing laboratory, without the need of the use of new technologies. Once the culture medium is manufactured, its use by any food and beverage industry or quality control laboratory is immediate, since it does not require specialized operators other than the ones in charge of routine microbiological analyses.

Therefore, one of the objectives of the present invention consists in providing the food industry with a procedure that allows the isolation, differential detection and enumeration of yeasts of the *Dekkera* and *Brettanomyces* genera by the production of characteristic colonies, medium color change and production of a phenol-like aroma, thus avoiding the inconveniences of said yeasts identification after their isolation, object being accomplished using the culture medium and method according to the present invention, as described above.

Thus, the use of the medium according to the invention allows:
a) the detection and identification of contaminations in the food and beverage industry due to yeasts of the *Dekkera* and *Brettanomyces* genera, in every step of the manufacturing process, from the raw materials to the finished and stored product.
b) the definition of the critical control points, in order to establish control criteria suited to each one of these points in the food and beverage industry.

Further, the medium according to the invention is useful for inclusion in yeast identification galleries.

### Preferred Embodiments of the Invention

In a preferred embodiment, the present invention refers to a culture medium for the differential detection and enumeration of yeasts of the *Dekkera* and *Brettanomyces* genera which comprises "Yeast Nitrogen Base" as a nutrient base in an amount from 5 to 10 g/L, preferably 6.7 g/L, ethanol as a non-fermentable energy source in an amount from 32 to 96 g/L, preferably 48 g/L, *p*-cumaric acid as an aromatic compound (4-ethylphenol) promoting substrate, in an amount from 0.05 to 1.0 g/L, preferably 0.1 g/L, bromocresol green as an acid-base indicator with turning points in the acid range, an antibiotic that inhibits several species of yeasts (cycloheximide) in an amount from 0.004 to 0.1 g/L, preferably 0,01 g/L, and a bacterial growth inhibitor antibiotic.

In this embodiment of the invention, after culture medium inoculation with a sample containing yeasts of the *Dekkera* and *Brettanomyces* genera, which may be a previously isolated sample of these yeasts, or a mixed sample of yeasts and/or yeasts and bacteria, and incubation under advantageous growth conditions for these yeasts genera, after about 5 to 12 days, identification is possible by a culture medium color change, from blue to yellow, development of cream colored colonies and the characteristic phenol-like aroma.

The present invention is further illustrated by means of the following examples, which are intended only to exemplify and by no means limit the scope of the invention.

### Examples

### Example 1:

### Preparation of a culture medium according to the invention

The culture medium, object of the present invention, can be prepared using the following formulation (g/L): Yeast Nitrogen Base (6.7), as the nutrient base; ethanol (48), as the non-fermentable energy source and as an inhibitor for some of the yeasts; *p*-cumaric acid (0.1), as the phenol-like producing aroma substract; bromocresol green (0.022), previously dissolved in NaOH, as the acid-base indicator; cycloheximide (0.01), as the inhibitor antibiotic for some of the yeast species; chloramphenicol (0.1) and/or oxytetracycline (0.1), as the bacteria inhibitor antibiotic; and agar-agar (20), as the gelling agent. The culture medium is sterilized according to the following: the agar-agar is dissolved in 70% of the total needed water, the pH is adjusted to 5.4 with a strong acid, and the resulting solution is sterilized in an autoclave at 120°C, for 20 minutes. The other components are dissolved in the remaining of the demineralized water, the pH is adjusted to 5.4 with a strong acid, and the resulting solution is sterilized by filtration through a 0.22 µm pore diameter membrane. Both of the above solutions are then mixed together when the agar-agar solution reaches 50°C. The medium is then homogenized and dispensed into Petri dishes, allowing it to solidify prior to the inoculation.

### Example 2:

### Use of the culture medium object of the invention for the detection of yeasts of the Dekkera and Brettanomyces genera in wines

In this example, two wines suspected of having been altered were analyzed using the culture medium of Example 1. 20 ml samples of each wine were filtered under aseptic conditions, through 0,22 µm pore diameter cellulose acetate membranes. Each membrane was placed on the surface of a Petri dish containing the medium of the invention and incubated at 25°C. After 3 days it was possible to observe colonies in one of the dishes, along with the change of the medium color from blue to yellow; when the dish was opened, the presence of a phenol-like aroma was not detected. In the other dish no colonies were detect after 3 days. After 9 days the dish where the colonies had been observed maintained the same characteristics. In the other dish it was possible to observe small cream colored colonies, a color change of the medium from blue to yellow and a characteristic phenol-like aroma. Using the classical identification methods it was confirmed that the colonies developed in the dish that did not show a phenol-like aroma did not belong to the *Dekkera* or *Brettanomyces* genera, while those of the dish that showed the phenol-like aroma belonged to these genera.

### Example 3

### Use of the culture medium object of the invention for the enumeration of yeasts in wines where the presence of filamentous fungi is suspected

In this case, when the development of molds on the surface of the Petri dishes can shield or inhibit the outcome of yeasts colonies, the Most Probable Number enumeration technique is used, using test tubes with the liquid culture medium. Consequently, agar-agar is not used in the medium formulation, the medium being completely sterilized by filtration. In this instance, the presence of yeasts of the *Dekkera* or *Brettanomyces* genera is detected when there is a clouding of the medium, a turning of its color from blue to yellow, and the presence of a phenol-like aroma; therefore, all the test tubes that show these characteristics are considered positive, and all the others negative.

Although the present invention is described based on its preferred embodiments, it should be apparent to any person skilled in the art that variations and modifications within the scope of the appended claims are possible.

## Claims

1. A differential culture medium for the enumeration of food and beverage contaminant yeasts of the *Dekkera* and *Brettanomyces* genera, **characterized in that** it comprises a nutrient base, ethanol as the only energy source, *p*-cumaric acid, an acid-base indicator with turning points in the acid range, an inhibitor antibiotic for sensitive yeasts species, and optionally a bacteria growth inhibitor and agar-agar.

2. A culture medium according to claim **1**, **characterized in that** the present amount of ethanol is from 32 to 96 g/L, preferably 48 g/L.

3. A culture medium according to claim **1**, **characterized in that** the present amount of *p*-cumaric acid is from 0.05 to 1.0 g/L, preferably 0.1 g/L.

4. A culture medium according to claim 1, **characterized in that** the nutrient base is "Yeast Nitrogen Base", in amounts from 5 to 10 g/L, preferably 6.7 g/L.

5. A culture medium according to claim **1**, **characterized in that** the inhibitor antibiotic for some of the yeast species is cycloheximide, present in an amount from 0.004 to 0.1 g/L, preferably 0.01 g/L.

6. A culture medium according to claim **1**, **characterized in that** the pH indicator is bromocresol green, present in an amount of 0.022 g/L.

7. A culture medium according to claim **6**, **characterized in that** the medium pH is adjusted with a strong acid to a value between 4.8 and 6.0, preferably 5.4.

8. A culture medium according to claim **1**, **characterized in that** it additionally contains a bacteria growth inhibitor, preferably chloramphenicol and/or oxytetracycline, in amounts of 0.1 g/L, to detect and identify yeasts of the *Dekkera* and *Brettanomyces* genera in food and beverage products containing mixed populations of yeasts and bacteria.

9. A culture medium according to any one of the preceding claims, **characterized in that** it contains all the components except agar-agar, to detect and identify yeasts of the *Dekkera* and *Brettanomyces* genera in food and beverage products containing mixed populations of yeasts, bacteria and particularly filamentous fungi.

10. A differential culture medium for enumeration of food and beverage contaminant yeasts of the *Dekkera* and *Brettanomyces* genera, **characterized in that** it has the following composition: 5 to 10 g/L, preferably 6.7 g/L, of "Yeast Nitrogen Base"; 0.004 to 0.1 g/L, preferably 0.01 g/L, of cycloheximide; 0.05 to 1.0 g/L, preferably 0.1 g/L, of *p*-cumaric acid; 0.022 g/L of bromocresol green, or another acid-base indicator with similar turning points; 32 to 96 g/L, preferably 48 g/L, of ethanol; 0.1 g/L of chloramphenicol and/or 0.1 g/L of oxytetracycline, and 20 g/L of agar-agar, the pH of the medium being adjusted between 4.8 and 6.0, preferably 5.4, with a strong acid.

11. A differential culture medium for enumeration of food and beverage contaminant yeasts of the *Dekkera* and *Brettanomyces* genera according to the preceding claims, **characterized by** the sterilization of all the components is done by filtration, except for the agar-agar which is sterilized in autoclave; the addition under aseptic conditions to this solution, after agar-agar cooling and before it solidifies, of all the other components of the medium, previously sterilized by filtration; and the dispensing of the medium into Petri dishes so that it solidifies.

12. A process for the detection and/or identification of yeasts of the *Dekkera* and *Brettanomyces* genera, **characterized by** the use of a differential culture medium for enumeration of food and beverage contaminant yeasts of the *Dekkera* and *Brettanomyces* genera, comprising a nutrient base, ethanol as the only energy source, *p*-cumaric acid, an acid-base indicator with turning points in the acid range, an inhibitor antibiotic for some of the yeast species, and optionally a bacteria growth inhibitor and agar-agar.

13. A process for the detection and/or identification of yeasts of the *Dekkera* and *Brettanomyces* genera according to claim **12**, **characterized in that** the acid-base indicator is bromocresol green, and after inoculation of said medium with a sample containing yeasts of the *Dekkera* and *Brettanomyces* genera, and incubation for **5** to **12** days in adequate conditions for the growth of said yeasts, it is possible to detect the presence, and if needed the enumeration of said yeasts genera, by means of a medium color change, from blue to yellow, and development of cream colored colonies and a phenol-like aroma, characteristic of the yeasts of the *Dekkera* and *Brettanomyces* genera.

14. A process for the detection and/or identification of yeasts of the *Dekkera* and *Brettanomyces* genera according to claims **12** and **13**, **characterized in that** it is applied to the detection and enumeration of yeasts of the *Dekkera* and *Brettanomyces* genera in food and beverage products samples.

15. Use of a culture medium according to claims **1** to **11**, for inclusion in an identification gallery, together with other yeast identification tests.

16. Use of a culture medium according to claims **1** to **11** in an industry, particularly in the quality and process control in a food and beverage industry.

## Patentansprüche

1. Differentialmedium für die Auszählung von Lebensmittel und Getränke kontaminierenden Hefen der Arten *Dekkera* und *Brettanomyces,* **dadurch gekennzeichnet, dass** es eine Nährstoffbasis, Ethanol als einzige Energiequelle, *p*-Cumarsäure, einen Säure-Base-Indikator mit Wendepunkten im sauren Bereich, einen für empfindliche Hefearten antibiotischen Inhibitor und wahlweise einen das Bakterienwachstum hemmenden Inhibitor und Agar-Agar umfasst.

2. Kulturmedium gemäß Anspruch 1, **dadurch gekennzeichnet, dass** die vorhandene Menge Ethanol von 32 bis 96 g/l, vorzugsweise jedoch 48 g/l beträgt.

3. Kulturmedium gemäß Anspruch 1, **dadurch gekennzeichnet, dass** die vorhandene Menge *p*-Cumarsäure von 0,05 bis 1,0 g/l, vorzugsweise jedoch 0,1 g/l beträgt.

4. Kulturmedium gemäß Anspruch 1, **dadurch gekennzeichnet, dass** die Nährstoffbasis "Yeast Nitrogen Base" ist und in einer Menge von 5 bis 10 g/l, vorzugsweise jedoch 6,7 g/l vorhanden ist.

5. Kulturmedium gemäß Anspruch 1, **dadurch gekennzeichnet, dass** der für einige Hefearten antibiotische Inhibitor Cycloheximid ist und in einer Menge von 0,004 bis 0,1 g/l, vorzugsweise jedoch 0,01 g/l vorhanden ist.

6. Kulturmedium gemäß Anspruch 1, **dadurch gekennzeichnet, dass** der pH -Indikator Bromcresol-grün ist und in einer Menge von 0,022 g/l vorhanden ist.

7. Kulturmedium gemäß Anspruch 6, **dadurch gekennzeichnet, dass** der pH-Wert des Mediums mit einer starken Säure auf einen Wert zwischen 4,8 und 6,0, vorzugsweise jedoch 5,4 eingestellt wird.

8. Kulturmedium gemäß Anspruch 1, **dadurch gekennzeichnet, dass** es zusätzlich einen das Bakterienwachstum hemmenden Inhibitor, vorzugsweise Chloramphenicol und/oder Oarytetracyclin, in einer Menge von 0,1 g/l enthält zum Nachweis und zur Identifikation von Hefen der Arten *Dekkera* und *Brettanomyces* in Lebensmitteln und Getränken, die gemischte Populationen von Hefen und Bakterien enthalten.

9. Kulturmedium gemäß einem der vorstehenden Ansprüche, **dadurch gekennzeichnet, dass** es sämtliche Bestandteile mit Ausnahme von Agar-Aagar enthält, zum Nachweis und zur Identifikation von Hefen der Arten *Dekkera* und *Brettanomyces* in Lebensmitteln und Getränken, die gemischte Populationen von Hefen, Bakterien und insbesondere Fadenpilzen enthalten.

10. Differentialmedium für die Auszählung von Lebensmittel und Getränke kontaminierenden Hefen der Arten *Dekkera* und *Brettanomyces,* **dadurch gekennzeichnet, dass** es folgende Zusammensetzung hat: 5 bis 10 g/l, vorzugsweise jedoch 6,7 g/l "Yeast Nitrogen Base"; 0,004 bis 0,1 g/l, vorzugsweise jedoch 0,01 g/l Cycloheximid; 0,05 bis 1.0 g/l, vorzugsweise jedoch 0,1 g/l *p*-Cumarsäure; 0,022 g/l Bromcresol-grün bzw. einen sonstigen Säure-Base-Indikator mit ähnlichen Wendepunkten; 32 bis 96 g/l, vorzugsweise jedoch 48 g/l Ethanol; 0,1 g/l Chloramphenicol und/oder 0,1 g/l Oxytetracyclin und 20 g/l Agar-Aagar, wobei der pH-Wert des Mediums mit einer starken Säure auf einen Wert zwischen 4,8 und 6,0, vorzugsweise jedoch auf 5,4 eingestellt wird.

11. Differentialmedium für die Auszählung von Lebensmittel und Getränke kontaminierenden Hefen der Arten *Dekkera* und *Brettanomyces* gemäß den vorstehenden Ansprüchen, **dadurch gekennzeichnet, dass** die Sterilisation sämtlicher Bestandteile durch Filtration erfolgt, mit Ausnahme des Agar-Agar, das in einem Autoklaven sterilisert wird; dass sämtliche sonstigen vorher durch Filtration sterilisierten Bestandteile des Mediums dieser Lösung nach Abkühlen und vor Erstarren des Agar-Agars unter aseptischen Bedingungen zugegeben werden und dass das Medium zum Erstarren in Petrischalen gegossen wird.

12. Verfahren zum Nachweis und/oder zur Identifikation von Hefen der Arten *Dekkera* und *Brettanomyces,* **dadurch gekennzeichnet, dass** ein Differentialmedium zur Auszählung von Lebensmittel und Getränke kontaminierenden Hefen der Arten *Dekkera* und *Brettanomyces* benutzt wird, das eine Nährstoffbasis, Ethanol als einzige Energiequelle, *p*-Cumarsäure, einen Säure-Base-Indikator mit Wendepunkten im sauren Bereich, einen für einige Hefearten antibiotischen Inhibitor und wahlweise einen das Bakterienwachstum hemmenden Inhibitor und Agar-Agar umfasst.

13. Verfahren zum Nachweis und/oder zur Identifikation von Hefen der Arten *Dekkera* und *Brettanomyces* gemäß Anspruch 12, **dadurch gekennzeichnet, dass** der Säure-Base-Indikator Bromcresol-grün ist und dass es nach Inokulation des genannten Mediums mit einer Hefen der Arten *Dekkera* und *Brettanomyces* enthaltenden Probe und Inkubation von 5 bis 12 Tagen unter für das Wachstum der genannten Hefen geeigneten Bedingungen möglich ist, das Vorhandensein der genannten Hefearten durch eine Farbänderung des Mediums von blau nach gelb und die Entwicklung cremefarbener Kolonien und eines für die Hefearten *Dekkera* und *Brettanomyces* charakteristischen Phenol-Geschmacks nachzuweisen und sie gegebenenfalls auszuzählen.

14. Verfahren zum Nachweis und/oder zur Identifikation von Hefen der Arten *Dekkera* und *Brettanomyces* gemäß Anspruch 12 und 13, **dadurch gekennzeichnet, dass** es zum Nachweis und zur Auszählung der Hefen der Arten *Dekkera* und *Brettanomyces* in Lebensmittel- und Getränkeproben verwendet wird.

15. Verwendung eines Kulturmediums gemäß den Ansprüchen 1 bis 11 zur Aufnahme in eine Identifikationskartei zusammen mit anderen Tests zur Identifikation von Hefen.

16. Verwendung eines Kulturmediums gemäß den Ansprüchen 1 bis 11 in der Industrie, insbesondere in der Qualitäts- und Verfahrenskontrolle in der Lebensmittel- und Getränkeindustrie.

## Revendications

1. Un milieu de culture spécifique pour le dénombrement des levures contaminantes des genres *Dekkera* et *Brettanomyces* dans les aliments et les boissons, **caractérisé en ce qu'**il comprend une base nutritive, de l'éthanol comme unique source d'énergie, de l'acide p-cumarique, un indicateur acide-base dont les points de virage se situent dans la zone acide, un antibiotique inhibiteur pour les espèces de levures sensibles, et, facultativement, un inhibiteur de croissance de bactéries et de l'agar-agar.

2. Un milieu de culture selon la revendication 1, **caractérisé en ce que** la teneur en éthanol présent est comprise entre 32 et 96 g/l, de préférence 48 g/l.

3. Un milieu de culture selon la revendication 1, **caractérisé en ce que** la teneur en acide p-cumarique présent est comprise entre 0,05 et 1,0 g/l, de préférence 0,1 g/l.

4. Un milieu de culture selon la revendication 1, **caractérisé en ce que** la base nutritive est une « base azotée de levure », en quantité variant entre 5 et 10 g/l, de préférence 6,7 g/l.

5. Un milieu de culture selon la revendication 1, **caractérisé en ce que** l'antibiotique inhibiteur pour certaines espèces de levures est le cycloheximide, en quantité variant entre 0,004 et 0,1 g/l, de préférence 0,01 g/l.

6. Un milieu de culture selon la revendication 1, **caractérisé en ce que** l'indicateur de pH est le vert de bromocrésol , dont la teneur est de 0,022 g/l.

7. Un milieu de culture selon la revendication 6, **caractérisé en ce que** le pH moyen est ajusté, à l'aide d'un acide fort, à des valeurs comprises entre 4,8 et 6,0, de préférence 5,4.

8. Un milieu de culture selon la revendication 1, **caractérisé par** la présence additionnelle d'un inhibiteur de croissance de bactéries, de préférence le chloramphénicol et/ou l'oxytétracycline, d'une quantité de 0,1 g/l, permettant la détection et l'identification des levures des genres *Dekkera* et *Brettanomyces* dans les produits alimentaires et les boissons contenant des populations mixtes de levures et de bactéries.

9. Un milieu de culture selon n'importe laquelle des revendications précédentes, **caractérisé en ce qu'**il contient tous les composants, à l'exception de l'agar-agar, permettant la détection et l'identification des levures des genres *Dekkera* et *Brettanomyces* dans les produits alimentaires et les boissons contenant des populations mixtes de levures, de bactéries et, en particulier, de champignons filamenteux.

10. Un milieu de culture spécifique pour le dénombrement des levures contaminantes des genres *Dekkera* et *Brettanomyces* dans les aliments et les boissons, **caractérisé par** la la composition suivante: 5 à 10 g/l, de préférence 6,7 g/l, de "base azotée de levure"; 0,004 à 0,1 g/l, de préférence 0,01 g/l, de cycloheximide; 0,05 à 1,0 g/l, de préférence 0,1 g/l, d'acide p-cumarique; 0,022 g/l de vert de bromocrésol ou de tout autre indicateur acide-base possédant des points de virage similaires; 32 à 96 g/l, de préférence 48 g/l, d'éthanol; 0,1 g/l de chloramphénicol et/ou 0,1 g/l d'oxytétracycline, et 20 g/l d'agar-agar, le pH du milieu étant ajusté à des valeurs comprises entre 4,8 et 6,0, de préférence 5,4, à l'aide d'un acide fort.

11. Un milieu de culture spécifique pour le dénombrement des levures contaminantes des genres *Dekkera* et *Brettanomyces* dans les aliments et les boissons, selon les revendications précédentes, **caractérisé par** la stérilisation effectuée par filtration de tous les composants, à l'exception de l'agar-agar qui, lui, est stérilisé dans un autoclave; l'ajout à cette solution, dans des conditions aseptiques, après le refroidissement de l'agar-agar et avant sa solidification, de tous les autres composants du milieu préalablement stérilisés par filtration; et la préparation du milieu dans des boîtes de Petri afin qu'il se solidifie.

12. Un procédé de détection et/ou d'identification des levures des genres *Dekkera* et *Brettanomyces,* **caractérisé par** l'utilisation d'un milieu de culture spécifique pour le dénombrement des levures contaminantes des genres *Dekkera* et *Brettanomyces* dans les aliments et les boissons, comprenant une base nutritive, de l'éthanol comme unique source d'énergie, de l'acide p-cumarique, un indicateur acide-base dont les points de virage se situent dans la zone acide, un antibiotique inhibiteur pour certaines espèces de levures, et, facultativement, un inhibiteur de croissance de bactéries et de l'agar-agar.

13. Un procédé de détection et/ou d'identification des levures des genres *Dekkera* et *Brettanomyces,* selon la revendication 12, **caractérisé en ce que** l'indicateur acide-base est le vert de bromocrésol, et que, après inoculation du milieu en question par un échantillon contenant des levures des genres *Dekkera* et *Brettanomyces* et incubation pendant 5 à 12 jours dans des conditions appropriées pour la croissance des levures en question, il est possible de détecter la présence, et, si nécessaire, le dénombrement des genres des levures en question, au moyen d'un changement de couleur du milieu, passant du bleu au jaune, du développement de colonies de couleur crème et d'une odeur ressemblant à celle du phénol, caractéristique des levures des genres *Dekkera* et *Brettanomyces*.

14. Un procédé de détection et/ou d'identification des levures des genres *Dekkera* et *Brettanomyces*, selon les revendications 12 et 13, **caractérisé en ce qu'**il est utilisé dans la détection et le dénombrement des levures des genres *Dekkera* et *Brettanomyces* dans des échantillons de produits alimentaires et de boissons.

15. L'utilisation d'un milieu de culture, selon les revendications 1 à 11, pour inclusion dans une galerie d'identification, en même temps que d'autres tests d'identification de levures.

16. L'utilisation d'un milieu de culture, selon les revendications 1 à 11, dans l'industrie, en particulier pour la qualité et le contrôle du processus dans l'industrie agro-alimentaire et des boissons.
